# EUROPEAN PATENT APPLICATION

(11) **EP 4 535 005 A1**
(43) Date of publication of application: **09.04.2025**
(21) Application number: 23811366.6
(22) Date of filing: 09.02.2023
(51) Int. Cl.: G01N 35/00, G01N 33/48, G01F 23/292

(54) **BIOLOGICAL SAMPLE MEASUREMENT DEVICE**

(30) Priority: 24.05.2022 JP 2022084300
(71) Applicant: HITACHI HIGH-TECH CORPORATION, Tokyo 105-6409 (JP)
(72) Inventor: SAO Mayu, Tokyo 100-8280 (JP); KAKUNO Masahito, Tokyo 105-6409 (JP)
(74) Representative: Mewburn Ellis LLP
(86) International application number: PCT/JP2023/004335
(87) International publication number: WO 2023/228485

(57) **Abstract**

An object of the disclosure is to provide a technique in which when measuring a biological sample stored in a container, to which a label is attached, and separated into a plurality of component regions, a target portion that is a measurement target can be accurately specified regardless of an orientation of the label without using a large-sized imaging mechanism. A biological sample measuring device according to the disclosure specifies an upper surface boundary and a lower surface boundary of a measurement target portion by specifying an intermediate point of luminance values of a captured image, and applies a correction amount of a meniscus width to a position of the specified upper surface boundary (see FIG. 1).

## Description

### Technical Field

The present disclosure relates to a device that measures a biological sample separated into a plurality of component regions.

### Background Art

For the purpose of improving efficiency of clinical tests such as blood tests, there is a demand for a technique for automating a related-art liquid amount checking operation of a biological specimen before opening (before aliquoting) performed by visual checking. Particularly, a biological sample such as a blood specimen before aliquoting is separated into a plurality of layers by centrifugal separation or the like, and a technique for measuring only a liquid amount of a sample that is an analysis target is required. In addition, since a barcode label for identification or a pre-label attached to a blood collection tube and shipped may be attached to the biological sample before the aliquoting, there is a demand for a technique capable of performing measurement in a state where the labels are attached.

With such a biological sample measuring device, for example, PTL 1 discloses a liquid detection device in which infrared light is applied to a specimen to detect transmitted light by a line sensor, a boundary between a label and a serum region (measurement target region) is obtained based on a primary differential value thereof, and a serum amount of the specimen is measured.

In PTL 2, a signal of transmitted light is acquired by changing a light amount, so that analysis can be performed regardless of presence or absence of attenuation of the transmitted light due to the label. In addition, PTL 2 discloses a technique in which pulsed light having two wavelengths is emitted by performing switching in a time-division manner to a sample separated into a plurality of layers, and the transmitted light is measured while scanning the sample in a vertical direction, thereby detecting a height of a predetermined region of the sample separated into the plurality of layers.

### Citation List

### Patent Literature

PTL 1: JP2004-037320A
PTL 2: US2012/0013889

### Summary of Invention

### Technical Problem

In the technique described in PTL 1, for a biological sample including a plurality of components, by signal processing based on differential from a signal of infrared transmitted light, the serum amount is measured by detecting a position in an upper-lower direction where the label is attached and heights of upper and lower surfaces of the serum which is a measurement target. However, the signal processing of PTL 1 has a problem that under condition of a specimen in which the transmitted light is scattered by the label, such as a specimen in which the label is attached to a detector side, signals indicating the upper and lower surfaces of the serum are blurred due to the scattering, and detection accuracy decreases. Accordingly, a rotation mechanism for detecting and aligning an orientation of the label (an orientation of the specimen) is required, and it is difficult to reduce a size of the device.

The technique disclosed in PTL 2 relates to a liquid amount measurement technique in which for the biological sample including a plurality of components, a boundary of a measurement target region is accurately specified to measure a liquid amount based on signals of the transmitted light having two wavelengths with different absorptances for a measurement target. However, in PTL 2, it is necessary to perform measurement while vertically scanning a specimen (blood collection tube) in a longitudinal direction, and there is a problem that it is difficult to reduce a size of the device due to a scanning mechanism.

The disclosure has been made in view of the problem described above, and an object thereof is to provide a technique in which when measuring a biological sample stored in a container, to which a label is attached, and separated into a plurality of component regions, a target portion that is a measurement target can be accurately specified regardless of an orientation of the label without using a large-sized imaging mechanism.

### Solution to Problem

A biological sample measuring device according to the disclosure specifies an upper surface boundary and a lower surface boundary of a measurement target portion by specifying an intermediate point of luminance values of a captured image, and applies a correction amount of a meniscus width to a position of the specified upper surface boundary.

### Advantageous Effects of Invention

According to a biological sample measuring device of the disclosure, when measuring a biological sample stored in a container to which a label is attached and separated into a plurality of component regions, even under an influence of scattering of transmitted light caused depending on an orientation of the label, it is possible to reduce variation in measurement depending on the orientation of the label. Accordingly, it is possible to reduce the influence of the scattering of the transmitted light due to the label and improve analysis accuracy while realizing miniaturization without attaching a rotation mechanism for aligning the orientation of the label.

### Brief Description of Drawings

[FIG. 1] FIG. 1 is a configuration diagram of a biological sample measuring device 1 according to Embodiment 1.
[FIG. 2A] FIG. 2A is a diagram showing a principle for specifying a measurement target region.
[FIG. 2B] FIG. 2B is a diagram showing a principle for specifying the measurement target region.
[FIG. 3A] FIG. 3A shows an example in which a label is attached to a container of a biological sample 2.
[FIG. 3B] FIG. 3B shows an example in which the label is attached to the container of the biological sample 2.
[FIG. 4A] FIG. 4A is an example of a transmission image.
[FIG. 4B] FIG. 4B shows a result obtained by imaging the same simulated specimen by actually changing an orientation of the label (an orientation of the specimen) using illumination of a wavelength (1550 nm) having absorption in the measurement target region, and extracting an inclination of a boundary of a serum upper surface.
[FIG. 4C] FIG. 4C shows a result obtained by imaging the same simulated specimen by actually changing the orientation of the label (the orientation of the specimen) using the illumination of the wavelength (1550 nm) having absorption in the measurement target region, and extracting an inclination of the boundary of the serum upper surface.
[FIG. 5A] FIG. 5A is an example of a transmission image.
[FIG. 5B] FIG. 5B shows a result obtained by extracting a signal of the serum upper surface when imaging is performed by illumination with a wavelength of 970 nm.
[FIG. 6] FIG. 6 is a graph showing results obtained by attaching two labels 301 to one side of the specimen, in which FIGS. 4B and 4C are overlapped.
[FIG. 7A] FIG. 7A shows a method of detecting a center point of a luminance change of a one-dimensional signal.
[FIG. 7B] FIG. 7B shows a method of detecting the center point of the luminance change of the one-dimensional signal.
[FIG. 8] FIG. 8 is a flowchart showing a procedure in which an image processing unit 14 measures a liquid amount (serum liquid amount) in a measurement target region 21.
[FIG. 9] FIG. 9 is a flowchart showing details of S2.
[FIG. 10] FIG. 10 is a flowchart showing details of S23.
[FIG. 11] FIG. 11 is a flowchart showing details of S24.
[FIG. 12] FIG. 12 is a flowchart showing details of S3.
[FIG. 13A] FIG. 13A shows a simulation model in which a meniscus is simulated.
[FIG. 13B] FIG. 13B shows a result obtained by performing a simulation of transmitted light (one-dimensional signal in the vicinity of the serum upper surface) by a light beam tracing simulation using the model of FIG. 13A.

### Description of Embodiments

### <Embodiment 1>

A biological sample to be measured in Embodiment 1 of the disclosure is a specimen to which a pre-opening (pre-analysis) label is attached, and is assumed to be separated into a plurality of component layers (typically, one to three layers) depending on presence or absence of centrifugal separation. A measurement target is a portion that is an analysis target (that is an aliquoting target) by a biochemical analyzer or the like such as a plasma or a serum of a specimen separated into a plurality of layers.

It is known that when the label is attached to a detector side, specimen transmitted light is scattered due to the label on the specimen surface, and a boundary becomes blurred as compared with a case where the label is attached to an illumination side. That is, an inclination of a signal greatly differs depending on an orientation of the label. Accordingly, a method of detecting a serum boundary that prevents variation in a detection signal due to the orientation of the label is required.

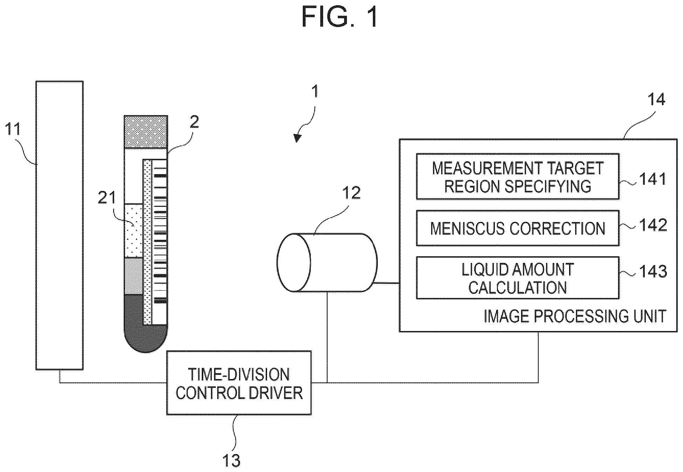

FIG. 1 is a configuration diagram of the biological sample measuring device 1 according to Embodiment 1 of the disclosure. The biological sample measuring device 1 is a device that measures the biological sample 2. The biological sample 2 is a sample configured as described above. The biological sample measuring device 1 specifies the measurement target region 21 of the biological sample 2, and measures a liquid amount thereof. The biological sample measuring device 1 includes a surface illumination light source 11, an area camera 12, a time-division control driver 13, and the image processing unit 14.

The surface illumination light source 102 switches a wavelength of emitted light between two wavelengths, and illuminates the biological sample 2 with the light. The light emitted from the surface illumination light source 11 can simultaneously illuminate two or more component layers (that is, across two or more component layers) that constitute the biological sample 2. In addition, regardless of the component layers, an upper surface of an uppermost layer (a boundary between the sample and an air layer) can be irradiated. Switching a wavelength of light does not necessarily require emitting light having only a single wavelength, and it is sufficient that a wavelength component having a highest intensity can be switched (wavelength λ1 = 1550 ± 100 nm, wavelength λ2 = 970 ± 100 nm, or the like). In the biological sample 2 having one or three component layers, when the number of the component layers is known before measurement, it is sufficient to use either of the two wavelengths, and there is no need to switch a wavelength. A reason and a method of selecting a wavelength will be described in a measurement principle to be described later.

The area camera 12 images surface illumination light transmitted through the biological sample 2, thereby generating a two-dimensional captured image of the biological sample 2. The area camera 12 has sensitivity characteristics that can detect light in a wavelength band emitted from the surface illumination light source 11. The area camera 12 can be implemented by, for example, an InGaAs camera.

The time-division control driver 13 switches the wavelength of the light emitted from the surface illumination light source 11 in a time-division manner. The time-division control driver 13 adjusts an exposure time (or gain) of the area camera 12 to a time suitable for the wavelength in synchronization with the switching of the wavelength. An imaging timing of the area camera 12 is controlled by the time-division control driver 13 in synchronization with the wavelength emitted from the surface illumination light source 11. The time-division control driver 13 may receive a processing result from the image processing unit 14 and control re-imaging according to the result.

The image processing unit 14 extracts a measurement target region (an image region of the specimen) from the captured image acquired by the area camera 12. An extraction principle will be described later. The image processing unit 14 includes a measurement target region specifying unit 141, a meniscus correction unit 142, and a liquid amount calculation unit 143. These operations will also be described later.

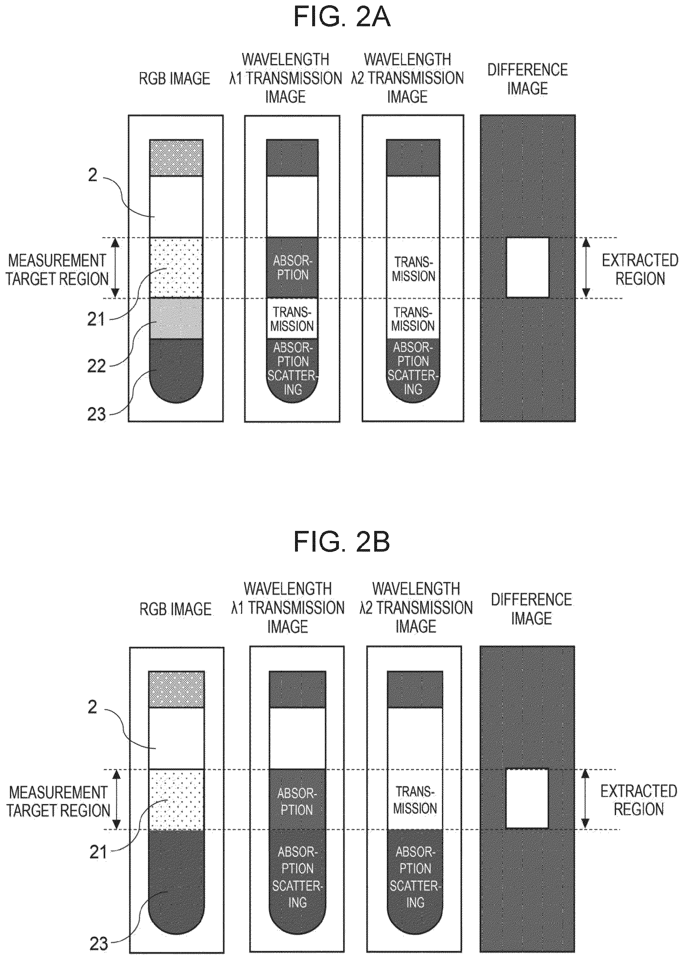

FIGS. 2A and 2B are diagrams showing a principle for specifying the measurement target region. In the configuration in FIG. 1, the area camera 12 captures a two-dimensional transmission image of the biological sample 2 as shown in FIGS. 2A and 2B.

FIG. 2A shows an example in which the biological sample 2 is separated into three layers. FIG. 2B shows an example in which the biological sample 2 is separated into two layers. A blood clot 23 is formed at a lower layer when the biological sample 2 is centrifugally separated. A separation material 22 is mixed to separate the blood clot 23 from the measurement target region 21.

When a first wavelength (wavelength 1) and a second wavelength (wavelength 2) emitted from the surface illumination light source 102 are compared, a transmittance when the wavelength 1 is transmitted through the blood clot 23 and a transmittance when the wavelength 2 is transmitted through the blood clot 23 are substantially the same. Therefore, a difference between a captured image of the blood clot 23 acquired using the wavelength 1 and a captured image of the blood clot 23 acquired using the wavelength 2 is very small. Similarly, for the separation material 22, since the transmittance of the wavelength 1 and the transmittance of the wavelength 2 are substantially the same, a difference between the two images is very small.

In contrast, a transmittance when the wavelength 1 is transmitted through the measurement target region 21 and a transmittance when the wavelength 2 is transmitted through the measurement target region 21 are greatly different. Therefore, a difference between a captured image of the measurement target region 21 acquired using the wavelength 1 and a captured image of the measurement target region 21 acquired using the wavelength 2 is remarkable. The difference is identified, whereby the measurement target region 21 can be extracted from the captured images.

Wavelength bands adopted as the wavelength 1 and the wavelength 2 need to be selected in advance such that a remarkable difference is generated between the wavelengths in the measurement target region 21, but almost no difference is generated in other portions as exemplified in FIGS. 2A and 2B. As long as the condition is satisfied, a specific numerical value of a wavelength may be optional. That is, at least a difference between the transmittance when the wavelength 1 is transmitted through the measurement target region 21 and the transmittance when the wavelength 2 is transmitted through the measurement target region 21 just needs to be larger than a difference between a transmittance when the wavelength 1 is transmitted through a region other than the measurement target region 21 and a transmittance when the wavelength 2 is transmitted through a region other than the measurement target region 21.

By using the measurement principle described above, when the biological sample 2 is separated into a plurality of component layers, the measurement target region 21 can be specified. The image processing unit 14 specifies the measurement target region 21 according to the principle. Although the number of component layers does not matter, a typical biological sample such as plasma or serum is separated into one to three layers. In either case, the measurement target region 21 can be accurately specified.

Regarding the biological sample 2 having one component layer and the biological sample 2 having three component layers, when the number of component layers is known in advance before the measurement, measurement can be performed using a transmission image having only one wavelength. In a case of three layers as shown in FIG. 2A, a wavelength having a difference in an absorptance between the measurement target region 21 and the separation material 22 or the air layer located above and below the measurement target region 21 may be selected. In a case of one layer, a wavelength having a difference between the air layer above the measurement target region 21 and an absorptance of the measurement target region 21 may be selected.

### <Embodiment 1: Problem of Transmitted Light Scattering due to Label>

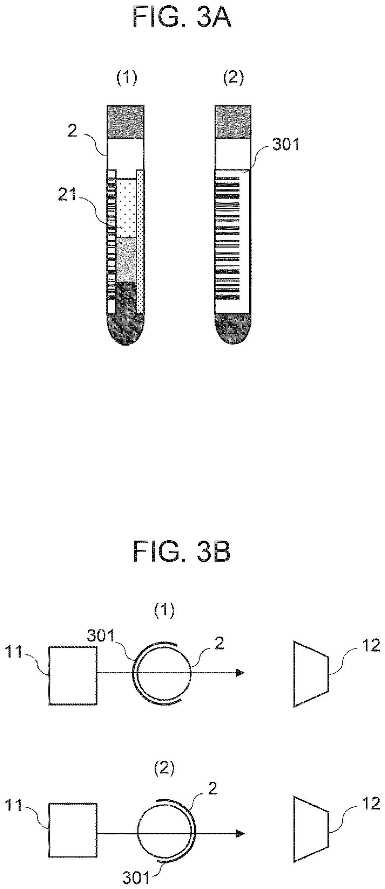

FIGS. 3A and 3B show an example in which a label is attached to a container of the biological sample 2. FIG. 3A is a side view of the container, and FIG. 3B is a top view thereof. Abarcode label, a pre-label (label 301), or the like may be attached to the container (for example, a blood collection tube) of the biological sample 2. Even in such a case, it is required to accurately specify the measurement target region 21. When a mechanism for controlling the orientation of the biological sample 2 is not provided, a positional relationship among the label 301, the surface illumination light source 11, and the area camera 12 varies for each biological sample 2. For example, there are a case where the label 301 is on an illumination side ((1) in FIG. 3B) and a case where the label 301 is on a side opposite to the illumination ((2) in FIG. 3B). In either case, it is required to specify the measurement target region 21 with the same accuracy.

When a transmission image is acquired by changing an orientation of the label 301 (changing an orientation of the biological sample 2) as shown in FIG. 3, it is known that the serum boundary is blurred when the label 301 is on the detector (area camera 12) side. This is because the light passing through the specimen is scattered by the label 301 on the specimen surface.

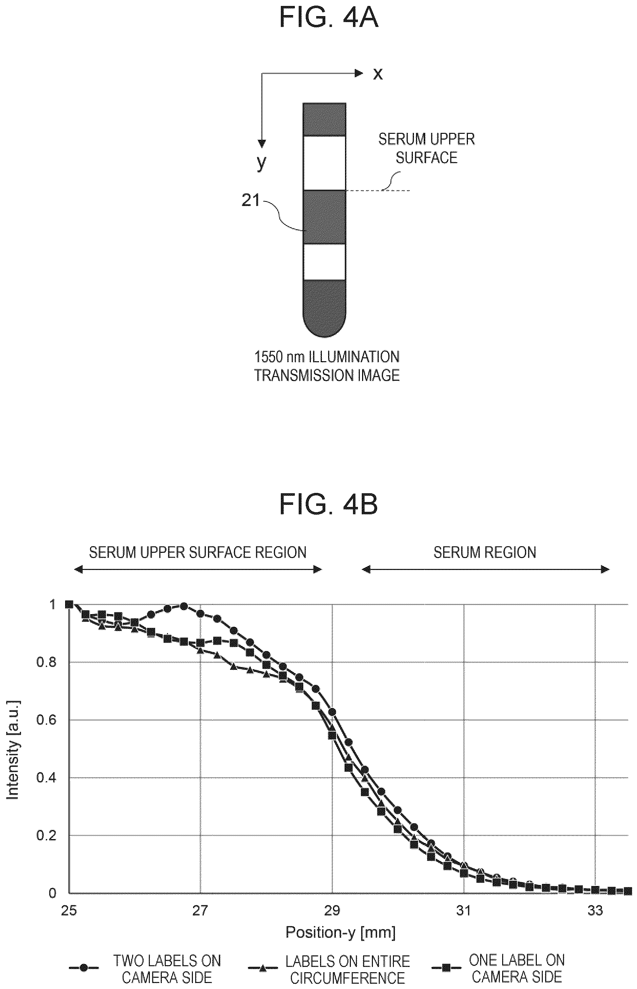

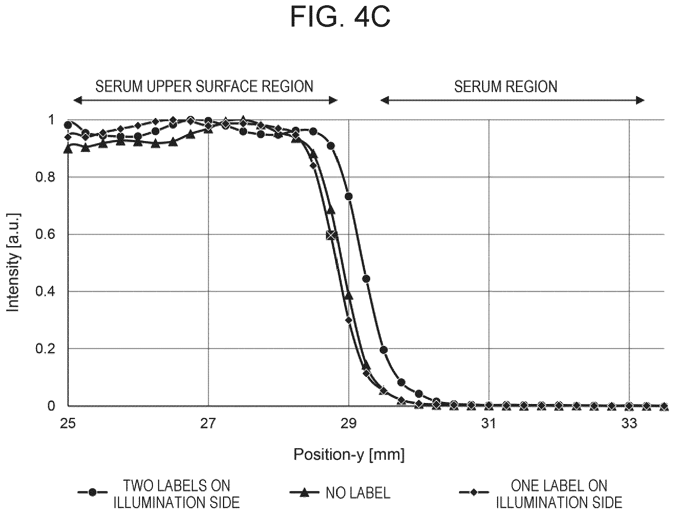

FIG. 4A is an example of the transmission image. FIG. 4B and 4C show a result obtained by imaging the same simulated specimen by actually changing the orientation of the label (the orientation of the specimen) using illumination of a wavelength (1550 nm) having absorption in the measurement target region, and extracting an inclination of a boundary of a serum upper surface. The image processing unit 14 extracts a width of 3 mm at a specimen center from an image captured at the wavelength of 1550 nm, and averages the extracted width in an x-direction to acquire a one-dimensional signal. The "label illumination side" corresponds to (1) in FIG. 3B, and the "label camera side" corresponds to (2) in FIG. 3B. FIG. 4B shows results obtained by measuring a specimen on which one label 301 is attached to the camera (area camera 12) side, a specimen on which two labels 301 are attached to the camera side, and a specimen on which (a total of two) labels 301 are attached to the entire circumference. FIG. 4C shows results obtained by measuring a specimen on which one label 301 is attached to the camera side, a specimen on which two labels 301 are attached to the camera side, and a specimen on which no label 301 is attached.

As shown in FIGS. 4B to 4C, it is known that an inclination of a signal in the vicinity of the serum boundary is greatly different between the case where the label 301 is on the camera side and the case where the label 301 is not on the camera side. Therefore, in the liquid amount measurement, it is necessary to detect a boundary position of the measurement target region 21 based on a measurement signal having a different inclination depending on the orientation of the label 301, that is, to reduce variation in a detected coordinate depending on the orientation of the label 301.

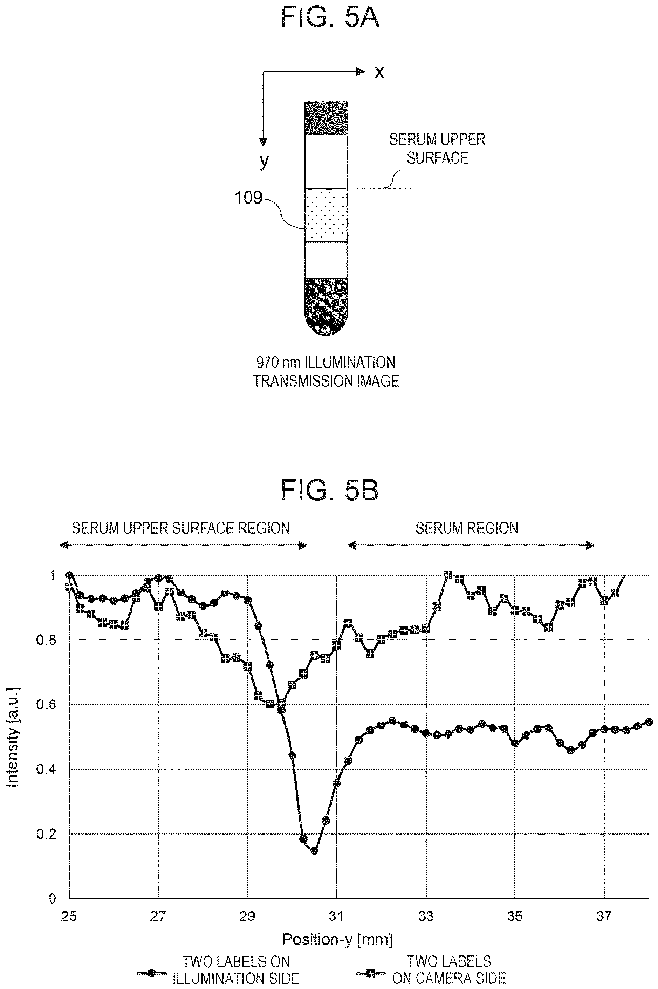

FIGS. 5A to 5B show a result obtained by extracting a signal of the serum upper surface when imaging is performed by illumination with a wavelength of 970 nm. The image processing unit 14 extracts a width of 3 mm at a specimen center from an image captured at the wavelength of 970 nm, and averages the extracted width in the x-direction as in FIGS. 4B to 4C to acquire a one-dimensional signal. FIG. 5B shows results of a case where two labels 301 are attached to the illumination (surface illumination light source 11) side and a case where two labels 301 are attached to the camera side.

In the case of the illumination with the wavelength of 970 nm, attenuation in a serum region is less than that in the case of the illumination with the wavelength of 1550 nm. In the case of the specimen having the label 301 on the illumination side, characteristic attenuation of transmitted light is observed in a meniscus region on the serum upper surface. This is because transmitted light is scattered and refracted due to a curved meniscus. On the other hand, when the label 301 is on the camera side, a shape of a dip is unclear. This is because the transmitted light is scattered due to the label on the specimen surface as in FIGS. 4B to 4C. That is, when the label is not on the camera side, a meniscus lower surface can be accurately measured by detecting a dip of the meniscus in the transmitted light having a wavelength of 970 nm. When the label is on the camera side, it is difficult to detect the meniscus lower surface because the dip becomes blurred due to an influence of scattering.

As described above, it is a problem to detect the meniscus lower surface under the condition that the scattering of the transmitted light due to the label 301 occurs, and to reduce the variation in the detection position depending on the orientation of the label 301.

### <Embodiment 1: Principle of Serum Boundary Signal Processing>

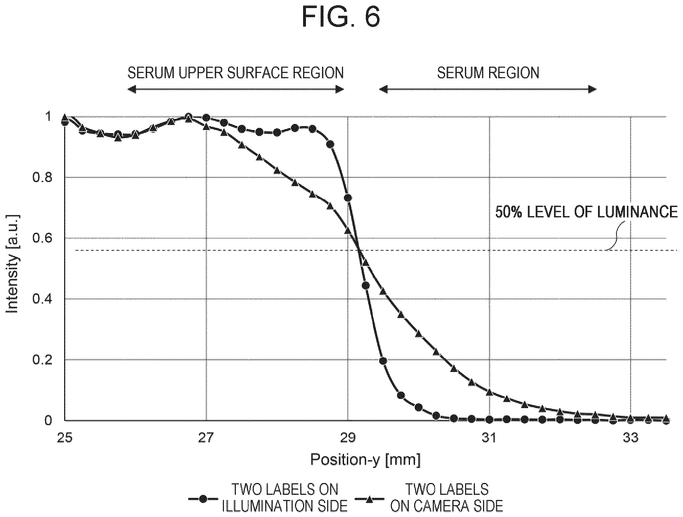

FIG. 6 is a graph showing results obtained by attaching two labels 301 to one side of the specimen, in which FIGS. 4B and 4C are overlapped. As shown in FIG. 6, the inclination of the signal greatly differs depending on the orientation of the label, but it is known that both have a characteristic of point symmetry at a point in the vicinity of a luminance of 50%. Accordingly, in the disclosure, the boundary of the measurement target region 21 is accurately detected regardless of the orientation of the label by using the symmetry and detecting a center point of the luminance change.

In an image region including the boundary (either the upper surface boundary or the lower surface boundary) of the measurement target region 21, a luminance value of the one-dimensional signal takes a maximum value (a left half region in FIG. 6) and a minimum value (a right half region in FIG. 6). Here, the luminance of 50% is a luminance value corresponding to a center point between the maximum value and the minimum value.

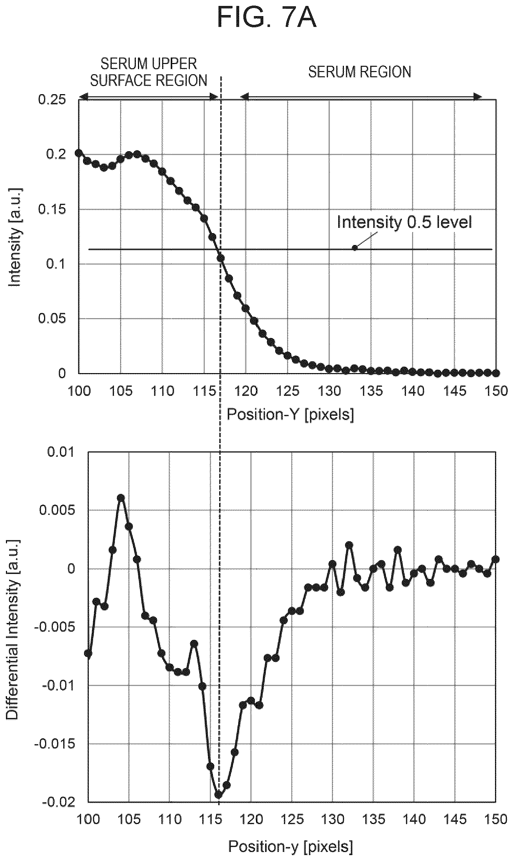

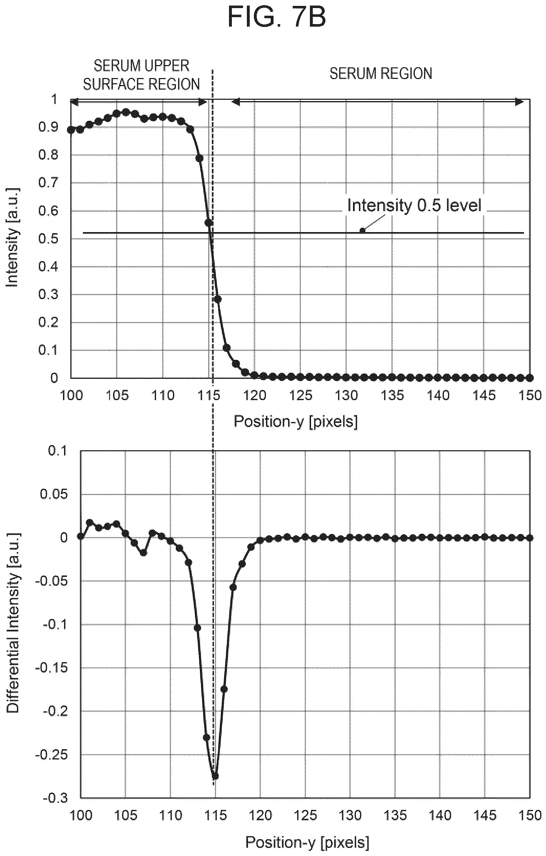

FIGS. 7A to 7B show a method of detecting the center point of the luminance change of the one-dimensional signal. The image processing unit 14 can detect an inflection point of the one-dimensional signal by detecting a peak of a primary differential of the one-dimensional signal (for example, detecting a zero cross point of a secondary differential of the one-dimensional signal), and specify the inflection point as the center point of the luminance change (that is, the boundary of the measurement target region 21). FIG. 7A shows a one-dimensional signal acquired by light having a wavelength of 1550 nm and a profile of a primary differential thereof when the label 301 is on the camera side. FIG. 7B shows a one-dimensional signal acquired by light having a wavelength of 1550 nm and a profile of a primary differential thereof when the label 301 is on the illumination side. It is known that peak coordinates of the primary differential correspond to coordinates in the vicinity of the luminance of 50%. Accordingly, the boundary of the measurement target region 21 can be specified by specifying a peak position of the primary differential of the one-dimensional signal. The same applies to both the upper surface boundary and the lower surface boundary.

As another method of detecting the center point of the luminance change of the one-dimensional signal, it is conceivable to specify a point at which the luminance value is at a 50% level of the maximum value by specifying the maximum value and the minimum value of the luminance change in the vicinity of the boundary of the measurement target region 21 and obtaining the center point between the maximum value and the minimum value. The same applies to both the upper surface boundary and the lower surface boundary. When a peak of the primary differential of the one-dimensional signal does not coincide with the center point, it is desirable to use this method.

For example, depending on variation or the like in the individual or type of the biological sample 2, the boundary of the measurement target region 21 may not be a center point between a maximum luminance and a minimum luminance in the vicinity of the boundary (not at 50% level of the luminance). Therefore, the image processing unit 14 can treat, as the boundary of the measurement target region 21, such a luminance level as to minimize the variation. For example, a luminance (for example, 60% level of luminance) slightly closer to the maximum luminance than the center between the maximum luminance and the minimum luminance in the vicinity of the boundary of the measurement target region 21 may be regarded as the boundary. In this case, instead of the center point between the maximum luminance and the minimum luminance, an intermediate point therebetween (in this example, 60% level of luminance) is used.

Similarly, even when the primary differential peak is used, a position obtained by further correcting the boundary position obtained by the inflection point of the luminance change may be regarded as a final boundary. For example, when a boundary position obtained using the primary differential peak and a boundary position obtained using the luminance center point are different from each other, a method of regarding an intermediate position between the two boundary positions as a boundary is considered.

Such correction of the boundary position can be performed by obtaining a correction amount in advance by experiments or the like and applying the correction amount to the boundary position obtained by the primary differential peak, the luminance center point, or the like. The processing of obtaining the correction amount can be performed, for example, in a calibration process before the measurement is performed.

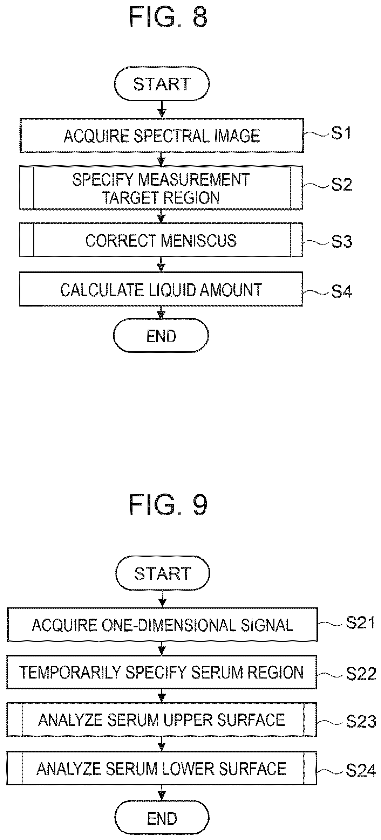

FIG. 8 is a flowchart showing a procedure in which the image processing unit 14 measures a liquid amount (serum liquid amount) in the measurement target region 21. The image processing unit 14 acquires transmission images having two wavelengths captured by the area camera 12 (S1). The measurement target region specifying unit 141 specifies the measurement target region 21 from the captured images having wavelengths (S2: details will be described later). The meniscus correction unit 142 applies the correction of the meniscus width to the boundary of the specified measurement target region 21 (S3: details will be described later). The liquid amount calculation unit 143 calculates the liquid amount in the measurement target region 21 (S4).

FIG. 9 is a flowchart showing details of S2. Hereinafter, each step in FIG. 9 will be described.

### (FIG. 9: Step S21)

The measurement target region specifying unit 141 acquires a one-dimensional signal of an acquired spectral image. The one-dimensional signal is obtained by extracting the vicinity of the center along a horizontal direction from the image of the biological sample 2 by a predetermined width and using a pixel value thereof. A width of the extracted central region is from 1 pixel to a width of the biological sample 2. A mean or a median of the pixel values in the central region is defined as the one-dimensional signal.

### (FIG. 9: Step S22)

The measurement target region specifying unit 141 acquires a difference between the one-dimensional signal of the transmission image acquired using the illumination with the wavelength of 970 nm and the one-dimensional signal of the transmission image acquired using the illumination with the wavelength of 1550 nm. The measurement target region specifying unit 141 temporarily specifies the measurement target region 21 based on the difference. A specific example is as described in FIGS. 2A to 2B.

### (FIG. 9: Step S23)

The measurement target region specifying unit 141 acquires a partial image in the vicinity of the upper surface boundary of the measurement target region 21 temporarily specified in S22, and specifies the upper surface boundary of the measurement target region 21 using the partial image. For example, a range of about ±5 mm of the boundary of the measurement target region 21 temporarily specified in S22 may be acquired as the partial image. By S23, noise resistance can be improved in the subsequent analysis. When the noise is small, this step can be omitted. The same applies to S24. Details of this step will be described later.

### (FIG. 9: Step S24)

The measurement target region specifying unit 141 acquires a partial image in the vicinity of the lower surface boundary of the measurement target region 21 temporarily specified in S22, and specifies the lower surface boundary of the measurement target region 21 using the partial image. A range of the partial image is, for example, about ±5 mm of the boundary of the measurement target region 21 temporarily specified in S22. Details of this step will be described later.

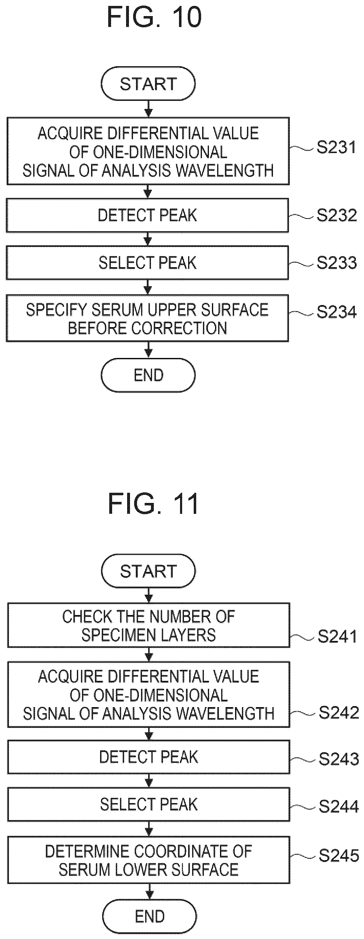

FIG. 10 is a flowchart showing details of S23. This flowchart implements the procedure described in FIGS. 6 to 7B. The serum upper surface is analyzed using a signal having a wavelength such as 1550 nm which is absorbed by the serum. Although an example in which a peak of the primary differential of the one-dimensional signal is used is shown in this flowchart, the maximum value and the minimum value of the luminance in the vicinity of the boundary may be used, or these values may be used in combination.

The measurement target region specifying unit 141 acquires a primary differential value of the one-dimensional signal (S231), and detects a peak of the primary differential value (S232). A smoothing filter such as a Gaussian filter may be applied to the one-dimensional signal before acquisition of a primary differential value or the primary differential value before a peak is detected (or may be applied to both). Accordingly, this has an effect of facilitating separation from noise. The measurement target region specifying unit 141 selects a peak having an absolute value equal to or larger than a preset threshold among the detected peaks (S233). For example, among the detected peaks, a peak closer to the serum side is selected based on the upper surface boundary of the measurement target region temporarily specified in S22 of FIG. 9. In addition, it may be checked whether the serum boundary is correctly detected by checking whether the luminance value of the one-dimensional signal at the coordinates before and after a candidate peak (about several millimeters) increases and decreases before and after the peak. Accordingly, the noise can be separated, and the accuracy is improved. Thus, the upper surface boundary of the measurement target region 21 can be specified (S234).

FIG. 11 is a flowchart showing details of S24. As described in FIGS. 2A to 2B, the wavelength at which a contrast occurs at the lower surface boundary of the measurement target region 21 is different depending on a substance present below the measurement target region 21. In the examples of FIGS. 2A to 2B, the transmission image of which wavelength is used differs depending on the number of layers constituting the biological sample 2. For example, the lower surface boundary is specified using the wavelength 2 in FIG. 2A, and the lower surface boundary is specified using the wavelength 1 in FIG. 2B. Therefore, the measurement target region specifying unit 141 switches the wavelength to be used according to the number of layers constituting the biological sample 2.

The measurement target region specifying unit 141 checks the number of layers constituting the biological sample 2 (S241). Specifically, a difference (contrast) between luminance values of partial regions crossing the lower surface boundary temporarily specified in S2 is acquired based on the captured images or the one-dimensional signals acquired at the wavelengths of 970 nm and 1550 nm. When the contrast is larger at 970 nm, the specimen is two layers, and the lower surface boundary is detected by analyzing the transmission image at 970 nm. When the contrast is larger at 1550 nm, the specimen is three layers, and the lower surface boundary is detected by analyzing the transmission image at 1550 nm. When the contrasts at both wavelengths are approximately the same (the contrast across the lower surface boundary is less than a threshold), the specimen is one layer, and the lower surface boundary is detected by analyzing the transmission image at the wavelength of 1550 nm. S242 to S245 are the same as S231 to S234.

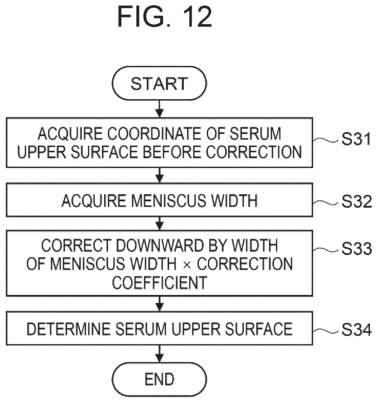

FIG. 12 is a flowchart showing details of S3. The meniscus correction unit 142 acquires upper surface boundary coordinates specified in S2 (S31). The meniscus correction unit 142 acquires a meniscus width (S32). The meniscus correction unit 142 acquires an assumed meniscus width in advance from a blood collection tube to be used, experimental data acquired in advance, or the like. Alternatively, when the label 301 is on the illumination side during the continuous measurement of the specimen, the meniscus width may be obtained from a signal of transmitted light at 970 nm (at this time, a characteristic dip is observed in the meniscus region). The meniscus correction unit 142 corrects a position of the upper surface boundary downward (in a direction toward a meniscus lower surface) by a width obtained by multiplying the meniscus width by a correction coefficient (S33). Examples of the correction coefficient will be described later. The meniscus correction unit 142 determines, as a final position of the upper surface boundary, the position of the upper surface boundary where the meniscus width is corrected (S34).

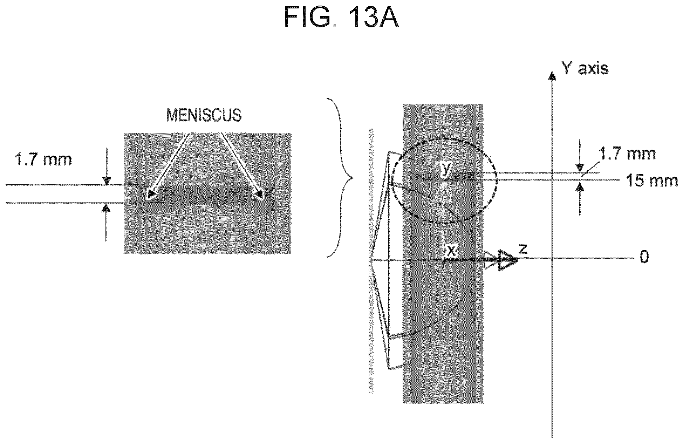

FIG. 13A shows a simulation model in which a meniscus is simulated. The upper surface boundary of the measurement target region 21 is a meniscus lower surface. Here, a position where a Y coordinate is 15 mm is the meniscus lower surface. The meniscus width is 1.7 mm.

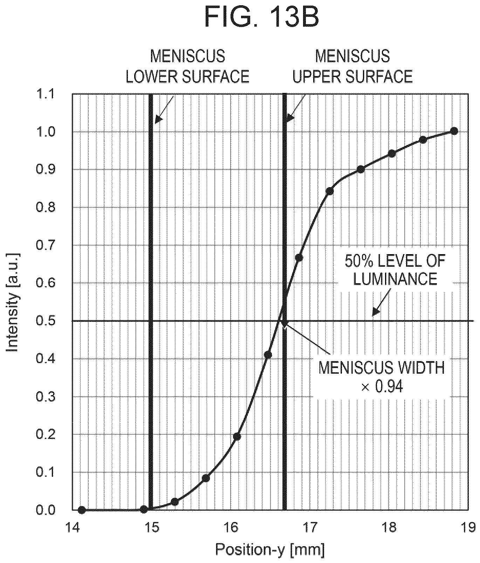

FIG. 13B shows a result obtained by performing a simulation of transmitted light (one-dimensional signal in the vicinity of the serum upper surface) by a light beam tracing simulation using the model of FIG. 13A. The upper surface boundary (50% level of the luminance in FIG. 13B) specified in S2 is shifted upward from the meniscus lower surface due to the influence of the meniscus. By correcting this shift, the meniscus correction unit 142 brings an estimated position of the upper surface boundary closer to the meniscus lower surface. The correction coefficient shown in FIG. 12 is defined by a ratio between a shift amount and the meniscus width. In FIG. 13B, it is shown by the simulation model of FIG. 13A that the coordinates of 50% level of the luminance are above the meniscus lower surface by the meniscus width × 0.94. Accordingly, the meniscus correction unit 142 calculates the correction amount by multiplying the meniscus width acquired in advance by the correction coefficient (the correction coefficient is 0.94 in the result of FIG. 13B), and corrects the position of the upper surface boundary downward toward the meniscus lower surface by applying the correction amount.

In view of the mechanism in which the estimated position of the upper surface boundary is shifted due to the meniscus, the correction coefficient is preferably a value from 0.5 to 1.0. The correction coefficient can be determined in advance by the simulation or calibration. The correction coefficient may be stored in advance in a storage unit provided in the biological sample measuring device 1, or may be acquired from a storage device provided in an external device. The correction coefficient may be obtained in advance for each type of the biological sample 2, and the correction coefficient may be used according to the type of the biological sample 2 during measurement.

According to the above procedure, the biological sample measuring device 1 can accurately specify the upper surface boundary and the lower surface boundary of the measurement target region 21 without adjusting the position (the position with respect to the surface illumination light source 11 or the position with respect to the area camera 12) of the label 301 attached to the biological sample 2.

### <Embodiment 2>

The biological sample measuring device 1 in Embodiment 1 can also be mounted as an additional option of an existing device in the existing device, or the biological sample measuring device 1 can also be used alone.

In Embodiment 1, the specific configuration (serum sample) of the biological sample 2 is assumed, and the specific example of the wavelength used for obtaining the transmission image is described. The disclosure is not limited thereto, and can be applied to other types of samples. That is, by appropriately selecting a wavelength for obtaining the transmission image as shown in FIGS. 2A to 2B based on absorbance characteristics of the sample, the wavelength can be applied to other samples.

The image processing unit 14 (and each functional unit provided in the image processing unit 14) can be implemented by hardware such as a circuit device where a function of the image processing unit 14 is provided, or can also be implemented by an arithmetic device such as a central processing unit (CPU) executing software where the function is provided.

In a method of acquiring a one-dimensional signal, the vicinity of a center along a horizontal direction may be extracted by a predetermined width from an image of the biological sample 2, and a mean or a median of pixel values may be obtained from the pixel values to obtain a one-dimensional signal, or each pixel row of two-dimensional images of the biological sample 2 may be treated as a one-dimensional signal. Analysis processing shown in FIG. 9 may be performed in units of pixel rows. When the analysis processing is performed in units of pixel rows, the mean or the median of the boundary coordinates determined in each pixel row is obtained to determine an upper surface and a lower surface.

### Reference Signs List

1: biological sample measuring device
11: surface illumination light source
12: area camera
13: time-division control driver
14: image processing unit
2: biological sample
21: measurement target region

## Claims

1. A biological sample measuring device that measures a biological sample separated into a plurality of component regions, the biological sample measuring device comprising:
a light source configured to irradiate the biological sample with first light having a first wavelength component and second light having a second wavelength component;
an imaging device configured to generate a captured image of the biological sample using the first and the second light transmitted through the biological sample; and
an image processing unit configured to specify, from the captured image, a target portion which is a measurement target in the component region, wherein
the image processing unit acquires a luminance value of the first light along a height direction of the target portion from the captured image acquired using the first light, and specifies a position of an upper surface boundary of the target portion along the height direction by specifying an intermediate point of a change in the luminance value of the first light in a first partial region of the captured image,
the image processing unit acquires a luminance value of the first light or the second light along the height direction from the captured image, and specifies a position of a lower surface boundary of the target portion along the height direction by specifying an intermediate point of a change in the luminance value of the first light or the second light in a second partial region of the captured image, and
the image processing unit corrects the specified position of the upper surface boundary by applying a correction amount for correcting a meniscus width of the target portion to the specified position of the upper surface boundary.

2. The biological sample measuring device according to claim 1, wherein
the image processing unit temporarily specifies the positions of the upper surface boundary and the lower surface boundary along the height direction from the captured image acquired using the first light and the second light,
the image processing unit specifies the position of the upper surface boundary along the height direction by specifying the intermediate point of the change in the luminance value of the first light in the first partial region including the temporarily specified upper surface boundary, and
the image processing unit specifies the position of the lower surface boundary along the height direction by specifying the intermediate point of the change in the luminance value of the first light or the second light in the second partial region including the temporarily specified lower surface boundary.

3. The biological sample measuring device according to claim 2, wherein
the imaging device generates a first captured image of the biological sample by using the first wavelength component of the first light,
the imaging device generates a second captured image of the biological sample by using the second wavelength component of the second light,
the image processing unit calculates a difference between a portion generated using the first wavelength component in the first captured image and a portion generated by using the second wavelength component in the second captured image, and
the image processing unit temporarily specifies the positions of the upper surface boundary and the lower surface boundary by specifying a portion at which the difference is equal to or larger than a threshold.

4. The biological sample measuring device according to claim 1, wherein
the imaging device is an area camera that generates a two-dimensional image of the biological sample as the captured image by using the first or second light transmitted through the biological sample, and
the image processing unit acquires, in a central region of the biological sample along a horizontal direction orthogonal to the height direction, a one-dimensional signal of the first light or the second light by statistically processing a pixel value of the two-dimensional image along the horizontal direction.

5. The biological sample measuring device according to claim 1, wherein
the image processing unit acquires a primary differential of the luminance value with respect to a position along the height direction, and specifies the position of the upper surface boundary or the position of the lower surface boundary by specifying a peak of the primary differential.

6. The biological sample measuring device according to claim 1, wherein
the image processing unit obtains a maximum value and a minimum value of the luminance value in a region including the upper surface boundary specified in the first partial region or the lower surface boundary specified in the second partial region, and
the image processing unit specifies, as the position of the upper surface boundary or the position of the lower surface boundary, a position in the height direction corresponding to the luminance value obtained by adding a predetermined ratio of a difference between the maximum value and the minimum value to the minimum value.

7. The biological sample measuring device according to claim 1, wherein
the image processing unit calculates the correction amount by multiplying the meniscus width by a correction coefficient, and
the image processing unit corrects the specified position of the upper surface boundary by applying the correction amount to the specified position of the upper surface boundary.

8. The biological sample measuring device according to claim 7, wherein
the image processing unit acquires the correction coefficient from a storage unit storing the correction coefficient corresponding to a type of the biological sample, and
the image processing unit corrects the position of the upper surface boundary using the correction coefficient acquired from the storage unit.

9. The biological sample measuring device according to claim 7, wherein
the correction coefficient is configured such that the correction amount is 0.5 times to 1.0 times the meniscus width.

10. The biological sample measuring device according to claim 3, wherein
the image processing unit compares a first contrast of the lower surface boundary temporarily specified using the first captured image with a second contrast of the lower surface boundary temporarily specified using the second captured image,
when the first contrast is larger than the second contrast by a predetermined value or more, the image processing unit acquires the luminance value of the first light along the height direction from the captured image acquired using the first light, and specifies the position of the lower surface boundary along the height direction by specifying an inflection point of the change in the luminance value of the first light in a region including the temporarily specified lower surface boundary, and
when the second contrast is larger than the first contrast by the predetermined value or more, the image processing unit acquires the luminance value of the second light along the height direction from the captured image acquired using the second light, and specifies the position of the lower surface boundary along the height direction by specifying an inflection point of the change in the luminance value of the second light in a region including the temporarily specified lower surface boundary.

11. The biological sample measuring device according to claim 10, wherein
when a difference between the first contrast and the second contrast is less than the predetermined value, the image processing unit acquires the luminance value of the first light along the height direction from the captured image acquired using the first light, and specifies the position of the lower surface boundary along the height direction by specifying the inflection point of the change in the luminance value of the first light in the region including the temporarily specified lower surface boundary.

12. The biological sample measuring device according to claim 1, wherein
the image processing unit measures a liquid amount of the target portion based on the specified position of the upper surface boundary and the specified position of the lower surface boundary.

13. The biological sample measuring device according to claim 1, wherein
a label is attached to a side surface of a container for accommodating the biological sample, and
the biological sample measuring device acquires the captured image by the imaging device without adjusting a positional relationship between the imaging device and the label.
